Europäisches Patentamt

European Patent Office

Office européen des brevets

(11) Veröffentlichungsnummer: **0 224 134**
**B1**

# (12) EUROPÄISCHE PATENTSCHRIFT

(45) Veröffentlichungstag der Patentschrift:
28.02.90

(21) Anmeldenummer: 86115758.4

(22) Anmeldetag: 13.11.86

(51) Int. Cl.⁴: **G01N 33/569**, G01N 33/577, C12Q 1/06

(54) Verfahren zur Quantifizierung von Zellpopulationen bzw. Subpopulationen sowie hierfür geeignetes Reagenz.

(30) Priorität: 19.11.85 DE 3541033

(43) Veröffentlichungstag der Anmeldung:
03.06.87 Patentblatt 87/23

(45) Bekanntmachung des Hinweises auf die Patenterteilung:
28.02.90 Patentblatt 90/9

(84) Benannte Vertragsstaaten:
AT BE CH DE ES FR GB GR IT LI LU NL SE

(56) Entgegenhaltungen:
GB-A- 2 122 345

(73) Patentinhaber: BOEHRINGER MANNHEIM GMBH,
Sandhofer Strasse 116, D-6800 Mannheim 31(DE)

(72) Erfinder: Schetters, Hartmut, Dr. rer. nat.,
Humboldtstrasse 5, D-8000 München 90(DE)
Erfinder: Endl, Josef, Dr. rer. nat., Hauptstrasse 17 a,
D-8031 Gliching(DE)
Erfinder: Albert, Winfried, Dr. phil., Moosstrasse 10,
D-8121 Pähl(DE)

ACTORUM AG

## Beschreibung

Die Erfindung betrifft ein Verfahren zur Quantifizierung von Zellpopulationen bzw. Subpopulationen, insbesondere Blutzellen, sowie ein hierfür geeignetes Reagens.

Das erfindungsgemäße Verfahren ist sonders geeignet zur Bestimmung von Lymphozyten, insbesondere T-Lymphozyten bzw. deren Subpopulationen, den T-Helfer- und T-Suppressor-Zellen.

T-Lymphozyten haben regulatorische Funktionen für das humorale und auch das zelluläre Immunsystem. Sie werden nach Entwicklungsstufen und Funktion eingeteilt. Bei T-Zellen müssen verschiedene Reifungsstufen, wie auch Untergruppen, unterschieden werden. Charakterisiert werden können diese durch Oberflächenantigene, die spezifisch sind für einzelne Entwicklungsstufen und für funktionelle Untergruppen. Klassifikationen sind möglich einerseit nach funktionellen Kriterien und andererseits durch serologische Diskriminierung. Durch die Vergleiche der verschiedenen Antigen-Zusammensetzungen lassen sich Schlüsse auf die Zellpopulationen und damit auf den augenblicklichen Immunstand des Spenders ziehen.

Die ruhenden T-Zellen müssen erst durch Antigen stimuliert werden, bevor sie funktionell aktiv sein können. So regen die T- Helferzellen die B-Lymphozyten an, zu proliferieren, zu differenzieren und Antikörper zu produzieren. Sie aktivieren ebenso aus der lymphoiden Zellreihe die Makrophagen und die T-Suppressorzellen, die zur zytotoxischen Reaktion gegen Zellen mit spezifischen Oberflächen-Antigenen angeregt werden.

T-Suppressor-Lymphozyten bewirken im aktivierten Zustand die Suppression der Antikörper-Produktion und die Reaktion von autologen T-Zellen in der gewünschten Lymphozyten-Kultur. Ebenso zeigen die T-Suppressor-Zellen zytotoxische Reaktion gegen die Targetzellen nach Sensitivierung mit HLA-Antigen tragenden Zellen (HLA = human leucocyte antigen.

Die üblichen Konzentrationen im Blut sowie der Quotient aus T-Helfer und T-Suppressor-Zellen liegen in den Bereichen:

| T-Lymphozyten (Gesamt) | 0,5 | – | 1,5 | × | $10^6$ Zellen/ml Blut |
|---|---|---|---|---|---|
| T-Suppressor | 0,15 | – | 0,45 | × | $10^6$ Zellen/ml Blut |
| T-Helfer | 0,3 | – | 0,9 | × | $10^6$ Zellen/ml Blut |
| T-Helfer/T-Suppressor | 1,2 | – | 3,9 | | |

Verschiedene Erkrankungen können Verschiebungen bei der Zusammensetzung der T-Zell-Subpopulationen bewirken, indem die Produktion einer Population vermindert oder verstärkt wird. Diese Veränderungen treten sehr früh im Krankheitsverlauf auf. Dazu gehören beispielsweise
- Krankheiten des Rheumakomplexes, Autoimmunerkrankungen (z. B. rheumatische Arthritis, systemischer Lupus Erythematosus)
- Infektionskrankheiten (z. B. AIDS, virale und fungale Infektionskrankheiten)
- Maligne T-Zellerkrankungen (z. B. Leukämien, Lymphome)

Die Ermittlung des Immunstatus, d. h. der Gesamtheit der T-Lymphozyten und der relativen Anteile der T-Helfer- und der T-Suppressorzellen, führt somit zu einem erheblichen diagnostischen Fortschritt.

Die bisherigen Bestimmungsmethoden für Zellpopulationen und Subpopulationen bestehen überwiegend im Anfärben der Zellen mit Antikörpern entsprechender Sprezifität, die mit Fluoreszenzfarbstoff markiert sind, und deren Auszählen unter dem Fluoreszenzmikroskop. Es ist offenkundig, daß dieses eine sehr aufwendige und personalintensive Methode ist und nur eine subjektive Beurteilung erlaubt.

Dieses Prinzip kann mit Hilfe eines "fluorescent activated cell sorter" (FACS) mechanisiert und objektiviert werden. Auf diese Weise kann zwar auf das mechanische Auszählen verzichtet werden. Die Methode ist jedoch mit einem erheblichen apparativen Aufwand verbunden und benötigt hochqualifiziertes Personal zur Bedienung.

Aufgabe der vorliegenden Erfindung war es, eine einfachere Methode bereitzustellen, mit der sowohl die Gesamtzahl von Zellpopulationen als auch von bestimmten Subpopulationen bestimmt werden kann. Die Aufgabe wird durch das erfindungsgemäße Verfahren gelöst.

Gegenstand der Erfindung ist ein Verfahren zur Quantifizierung von Zellpopulationen bzw. Subpopulationen, dadurch gekennzeichnet, daß
- die Probe mit der zu bestimmenden Zellpopulation mit markierten Antikörpern inkubiert wird, die spezifisch gegen charakteristische Oberflächenantigene der zu quantizierenden Zellpopulationen gerichtet sind,
- eine oder mehrere Standards mit bekannten, unterschiedlichen Partikelkonzentrationen mit denselben markierten Antikörpern in gleicher Weise inkubiert werden, wobei die Standardlösung aus Partikeln besteht, die in ihrem Sedimentationsverhalten mit den zu bestimmenden Zellen gleichen und die mit Molekülen beladen sind, die gegen den markierten Antikörper, bzw. einen Teil hiervon gerichtet sind,
- die Zellen der Probelösung sowie die Partikel der Standardlösungen von den überschüssigen markierten Antikörpern abgetrennt werden,

- die Menge der Markierung sowohl auf den Zellen als auch auf den Partikeln gemessen wird und
- durch Vergleich des Meßwertes aus der Probe mit den Meßwerten aus den Standardlösungen die Anzahl der zu bestimmenden Zellen in der Probe ermittelt wird.

Das erfindungsgemäße Verfahren ist allgemein brauchbar, um Zellpopulationen bzw. Subpopulationen zu quantifizieren. Besonders geeignet ist die Methode zur Quantifizierung von Blutzellen, insbesondere Lymphozyten. Besonders vorteilhaft hat sich das Verfahren bei der Bestimmung der Gesamtzahl der T-Zellen und der T-Helfer- und T-Suppressor-Subpopulationen erwiesen. Es ist damit eine zuverlässige und genaue Aussage über den momentanen Immunstatus für die Diagnose und Therapieverlaufskontrolle zu erzielen.

Als Antikörper, die gegen Antigene gerichtet sind, die charakteristisch für die zu bestimmende Zellpopulation sind (Zelloberflächenantigene), können sowohl polyklonale als auch manoklonale Antikörper eingesetzt werden. Es können die vollständigen Antikörper oder auch Antikörperfragmente verwendet werden. Besonders bevorzugt sind die monoklonalen Antikörper.

Zur Markierung der eingesetzten Antikörper stehen dem Fachmann verschiedene bekannte Methoden zur Verfügung. Es können hierbei alle üblichen Markierungsmittel verwendet werden, beispielsweise Radioisotope, Farbstoffe, Fluoreszenzfarbstoffe oder Enzyme. Im Sinne der vorliegenden Erfindung ist besonders die Markierung mit Enzymen bevorzugt. Als Markierungsenzyme können beispielsweise Peroxidase, alkalische Phosphatase, Glukoseoxidase oder besonders bevorzugt β-Galactosidase eingesetzt werden.

Der Test wird vorzugsweise als Antikörperbindungstest, vorzugsweise als solid phase Immunoassay durchgeführt. Als Festphase werden die zu bestimmenden Zellen bzw. die Partikel des Standards benutzt, die ähnliche Größe und Dichte bzw. ein ähnliches Sedimentationsverhalten besitzen wie die Zellen.

Es war eine besondere Schwierigkeit bei der Testentwicklung, einen Standard zu finden, der die Eigenschaften von Lymphozyten imitieren kann, so daß Lyomphozyten durch diesen ersetzt werden können. Der Standard soll die Vorzüge eines natürlichen Lymphozyten-Standards besitzen, ohne jedoch dessen Nachteile aufzuwerten:
- Lymphozyten sind nicht im benötigten Umfang verfügbar,
- Lymphozyten sind uneinheitlich, was zu einer mangelnden Reproduzierbarkeit von Charge zu Charge führt,
- Lymphozyten sind nicht ausreichend stabil, was Probleme hinsichtlich Haltbarkeit eines Lymphozyten-Standards führt.

Alle diese Nachteile konnten durch einen Standard mit künstlichen Partikeln überwunden werden.

Als solche Partikel werden kleine Kügelchen, die aus Glas oder Kunststoff bestehen können, bevorzugt. Besonders vorteilhaft sind Kunststoffpartikel, insbesondere Latexpartikel, die auf Methacrylat-Basis aufgebaut sind. Diese Partikel sollten vorzugsweise einen Durchmesser von 1 - 20 µm, bevorzugt 7 - 13 µm aufweisen.

An die Standardpartikel sind Moleküle (Proteine) gekoppelt, die gegen den markierten Antikörper bzw. einen Teil hiervon gerichtet sind. Es kann sich hierbei einmal um Proteine handeln, die dieselbe antigene Wirkung besitzen wie die Oberflächenantigene auf den zu bestimmenden Zellen. Die Partikel können jedoch auch Anti-Antikörper tragen, die gegen die markierten Antikörper, vorzugsweise gegen den $F_C$-Teil dieses Antikörpers, gerichtet sind. Schließlich können sich auf den Partikeln auch Antikörper befinden, welche die Markierung, beispielsweise im Falle einer Enzymmarkierung das Enzym, spezifisch erkennen.

Ein weiterer Gegenstand der Erfindung ist der Standard für die Quantifizierung von Zellpopulationen bzw. Subpopulationen, dadurch gekennzeichnet, daß er künstliche Partikel enthält, die in ihrem Sedimentationsverhalten den zu bestimmenden Zellen gleichen und die Moleküle tragen, die gegen den markierten Antikörper bzw. einen Teil hiervon gerichtet sind.

Zur Herstellung dieses Standards werden die Partikel zunächst aktiviert, beispielsweise mit Natriumperjodat. Anschließend wird ein Teil der aktivierten Partikel mit einem unspezifischen Antikörper, beispielsweise Schaf-IgG, nach bekannten Methoden gekoppelt. Der andere Teil der aktivierten Partikel wird mit dem spezifisch gegen den markierten Antikörper bzw. gegen einen Teil hiervon gerichteten Molekül verknüpft. Durch Mischen verschiedener Anteile dieser beiden verschieden markierten Partikelarten werden Standardlösungen hergestellt, die hinsichtlich ihrer Partikelzahl konstant sind, hinsichtlich der gebundenen spezifischen Moleküle jedoch variieren.

Die genaue Konzentration an spezifischen Molekülen in einem bestimmten Standard kann durch Vergleich mit einer bekannten Lymphozitenkonzentration, die durch konventionelles Auszählen in der Immunfluoreszenz oder mit Hilfe eines Zellsorters ermittelt worden ist, bestimmt werden. Die Bindungsfähigkeit der so kalibrierten Partikel entspricht einer bestimmten Zahl der zu bestimmenden Zellpopulation bzw. Subpopulation.

Der Standard kann als Lösung oder als Lyophilisat vorliegen. Das Lyophilisat muß vor dem Gebrauch in einem geeigneten Lösungsmittel, beispielsweise bidest. Wasser, resuspendiert werden.

Gegenstand der Erfindung ist ferner ein Reagenskit zur Durchführung des erfindungsgemäßen Verfahrens, dadurch gekennzeichnet, daß es

– den markierten Antikörper, der spezifisch gegen die zu bestimmende Zellpopulation gerichtet ist,
– eine oder mehrere Standards gemäß vorliegender Erfindung
– die für die Messung der Markierung erforderlichen Reagenzien sowie
– gegebenenfalls erforderliche weitere Hilfsmittel

enthält.

Auch die einzelnen Bestandteile liegen vorzugsweise in lyophilisierter Form vor. Sie müssen vor Gebrauch in einem geeigneten Lösungsmittel, beispielsweise bidest. Wasser, resuspendiert werden.

Die vorliegende Erfindung wird durch die folgenden Beispiele näher erläutert.

Beispiel 1:

Bestimmung der Gesamtzahl von T-Lymphozyten

1. Herstellung der Lösungen

a) Standardlösungen

Latexpartikel auf Methacrylatbasis (hergestellt gemäß DE-A 3 048 883) werden bei pH 5,0 mit Natriumperjodat aktiviert. Die aktivierten Latexpartikel werden in zwei Hälften geteilt. Die eine Hälfte wird mit unspezifischem Schaf-IgG, die andere Hälfte mit Schaf-IgG-anti-Maus-IgG gekoppelt. Durch Vermischen dieser beiden Chargen in verschiedenen definierten Verhältnissen werden drei Standardlösungen hergestellt, die einer Lyomphozyten-Konzentration von $0,45 \cdot 10^6$, $1,56 \cdot 10^6$ bzw. $3,36 \cdot 10^6$ Zellen/ml entsprechen. Die Lösungen werden lyophilisiert und sind in dieser Form über längere Zeit haltbar. Vor dem Gebrauch werden die Standards in bidest. Wasser resuspendiert (Eichstandardsuspensionen a, b und c).

b) Wasch-Puffer

1,47 g Dinatriumhydrogenphosphat ($Na_2HPO_4 \cdot 2H_2O$), 0,27 g Natriumdihydrogenphosphat ($NaH_2PO_4 \cdot H_2O$) und 8,77 g Natriumchlorid werden in bidest. Wasser gelöst und auf 1 1 Wasser aufgefüllt. Zu dieser Lösung werden Rinderserumalbumin, 0,2% (w/v), und Natriumazid, 0,1% (w/v), gegeben. Zur Lagerung wird lyophilisiert. Vor Gebrauch wird das Lyophilisat in bidest. Wasser resuspendiert.

c) Antikörper-Konjugat

Monoklonaler Antikörper CD 6 (M-T411, näher charakterisiert in: P. Rieber u.a. in Leucocyte typing (A. Bernard u.a., ED.), 1984, Seite 303-311, Springer Verlag, Berlin, New York) wird mit ß-D-Galactosidase gekoppelt und lyophilisiert. Pro Bestimmung werden 0,025 U, gelöst in Wasch-Puffer, eingesetzt.

d) Substrat-Lösung

60 mg Chlorphenolrot-β-D-galactosid werden in 30 ml Substrat-Puffer (100 mmol/l HEPES-Puffer (pH 7,1), 2 mmol/l Magnesiumaspartat, 1 % (w/v) Rinderserumalbumin und 0,1 % (w/v) Natriumazid) gelöst.

2. Probenvorbereitung

Die Blutprobe wird mit Ethylendiamintetraessigsäure-dinatriumsalz ($EDTA-Na_2.2H_2O$) vermischt. 7,5 ml von diesem frischen EDTA-Blut werden mit 15 ml Lyse-Puffer (155 mmol/l Ammoniumchlorid; 10 mmol/l Kaliumhydrogencarbonat; 0,1 mmol/l $EDTA-Na_2$) vermischt. Die Mischung wird 7 - 10 Minuten bei Raumtemperatur stehengelassen, wobei zwei- bis dreimal vorsichtig umgeschwenkt wird. Danach wird 10 Minuten bei 300 g zentrifugiert. Der Überstand mit den lysierten Erythrozyten wird abgesaugt (Pasteur-Pipette, Vakuum-Pumpe).

Das Leukozyten-Sediment wird aufgewirbelt (Vortex-Mischer), zur Lyse restlicher Erythrozyten wird nochmals mit 2 ml Lysepuffer resuspendiert. Die Suspension wird 5 Minuten stehengelassen, dabei gelegentlich umgeschwenkt. Danach wird 4 ml PBS (phosphate buffered saline)-Lösung (150 mmol/l Natriumchlorid, 10 mmol/l Natriumphosphat-Puffer, pH 7,3) zugegeben. Es wird erneut 10 Minuten bei 300 g zentrifugiert. Der Überstand wird abgesaugt.

Das Zellsediment wird aufgewirbelt und zweimal mit jeweils 5 ml PBS-Lösung gewaschen. Nach erneutem Zentrifugieren werden die Überstände sorgfältig abgesaugt. Das Sediment wird aufgewirbelt und mit 2 ml Resuspensions-Puffer (bestehend aus PBS-Lösung, Rinderserum Albumin, 0,2 % (w/v), Rinderserum Immunglobulin, 1 % (w/v), Natriumzid, 0,1 % (w/v)) aufgenommen. Diese Zellsuspension wird sofort weiterverarbeitet.

3. Durchführung der Messung

Wellenlänge: 578 nm
Temperatur: Raumtemperatur (20 - 25° C)
Halbmikroküvette: 1 cm Schichtdicke
Meßvolumen: 1 ml; Messung gegen Reagenzienleerwert
In Zentrifugenröhrchen mit konischem Boden werden pipettiert:

| | Standard | | | Probe |
|---|---|---|---|---|
| | a | b | c | |
| Eichstandard-Suspensionen a/b/c | 0,5 ml | 0,5 ml | 0,5 ml | – |
| Wasch-Puffer | 2,0 ml | 2,0 ml | 2,0 ml | – |

Es wird 10 Minuten bei 300 g zentrifugiert. Die Überstände werden sorgfältig abgesaugt. Die Sedimente werden aufgewirbelt und dann wie folgt pipettiert:

| | Standard | | | Probe |
|---|---|---|---|---|
| | a | b | c | |
| Wasch-Puffer | 0,2 ml | 0,2 ml | 0,2 ml | – |
| Zellsuspension (Probe aus 2.) | – | – | – | 0,2 ml |
| Antikörper-Konjugat | 0,1 ml | 0,1 ml | 0,1 ml | 0,1 ml |

Es wird gut gemischt, 45 Minuten bei Raumtemperatur (20 - 25° C) inkubiert und im 15 Minuten-Abstand kräftig aufgeschüttelt. Danach wird jeweils 2 ml Wasch-Puffer zugefügt, erneut vermischt, bei 300 g 10 Minuten zentrifugiert. Die Überstände werden sorgfältig abgesaugt, Sedimente aufgewirbelt. Es wird mehrmals mit jeweils 2 ml Wasch-Puffer gemischt, bei 300 g 10 Minuten zentrifugiert. Die Überstände werden wiederum sorgfältig abgesaugt, die Sedimente aufgewirbelt. Anschließend wird mit jeweils 1 ml Substratlösung Chlorphenolrot-β-Galactosid resuspendiert. Es wird gut gemischt, 30 Minuten bei Raumtemperatur (20 - 25° C) inkubiert, dabei wird nach 15 Minuten und 30 Minuten jeweils kräftig geschüttelt und 5 Minuten bei 700 g zentrifugiert. Die Extinktionen der klaren Überstände werden gegen die Substratlösung als Reagenzienleerwert gemessen.

4. Auswertung

Mit Hilfe der gemessenen Eichstandardwerte und den entsprechenden Konzentrationen wird eine Eichkurve erstellt. Daraus wird der dem Meßwert entsprechende Zellwert für die Probe direkt abgelesen.

Beispiel 2

Bestimmung der T-Helfer-Subpopulation

Die Vorbereitung der Probe, die Durchführung der Messung und die Auswertung erfolgen in der Beispiel 1 angegebenen Weise. Es werden lediglich als Eichstandardsuspensionen a, b und c Suspensionen verwendet, die einer T-Helfer-Konzentration von $0,20 \cdot 10^6$, $0,60 \cdot 10^6$ bzw. $2,08 \cdot 10^6$ Zellen/ml entsprechen. Ferner wird als Antikörperkonjugat ein Konjugat aus einem monoklonalen Antikörper CD 4 (M-T 151, näher charakterisiert in: a.a.O.) der spezifisch gegen Oberflächenantigene gerichtet ist, die für die T-Helfer-Subpopulation charakteristisch sind, und β-Galactosidase verwendet. Für jede Bestimmung werden 0,05 U Antikörper eingesetzt.
Aus den gemessenen Eichstandardwerten kann eine Eichgerade erstellt werden, aus der der dem Meßwert entsprechende Zellwert für die Probe direkt abgelesen werden kann.

Beispiel 3

Bestimmung der T-Suppressor-Subpopulation

Die Probenvorbereitung und die Durchführung der Messung wird in Analogie zu Beispiel 1 durchgeführt. Es wird lediglich als Antikörperkonjugat ein Konjugat aus β-Galactosidase und einem Antikörper verwendet, der spezifisch auf ein Oberflächenantigen der T-Suppressor-Zellen gerichtet ist, CD 8 (M-T 811, näher Charakterisiert in a.a.O.). Für jede Bestimmung werden 0,025 U Antikörper eingesetzt. Ferner Werden als Eichstandard suspension a, b und c drei verschieden konzentrierte Suspensionen verwendet, die einer T-Suppressor-Konzentration von $0,29 \cdot 10^6$, $0,62 \cdot 10^6$ bzw. $1,17 \cdot 10^6$ Zellen/ml entsprechen.

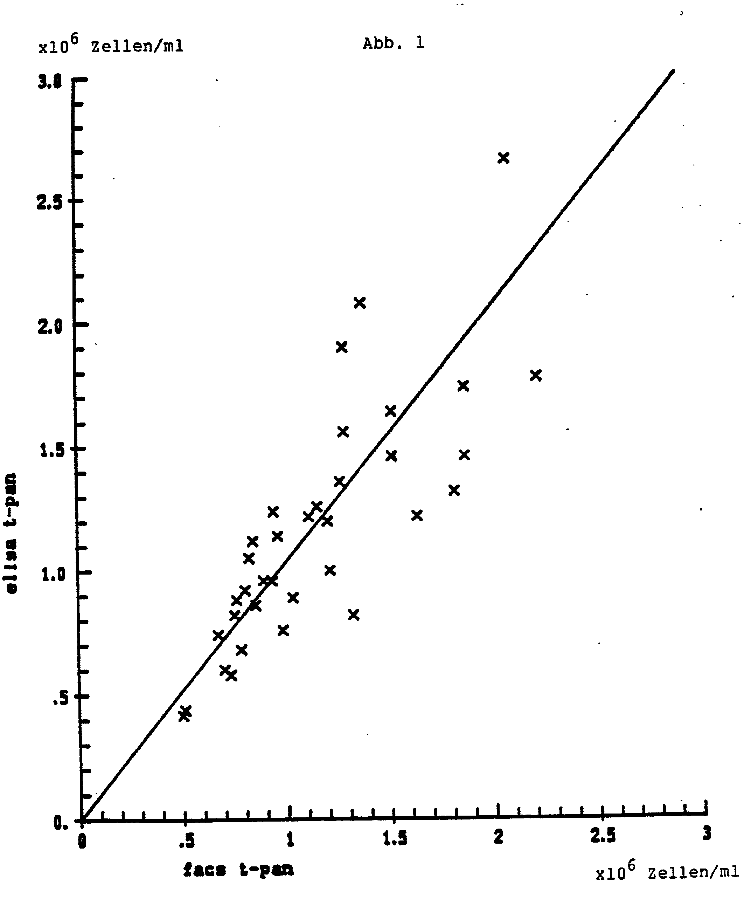

Die mit dem erfindungsgemäßen Reagens nach dem erfindungsgemäßen ELISA-Verfahren erhaltenen Ergebnisse korrelieren sehr gut mit Werten, die nach der bisher üblichen, wesentlich aufwendigeren Methode der mechanischen Auszählung erhalten werden (vgl. Abb.1, Abb. 2 und Abb. 3).

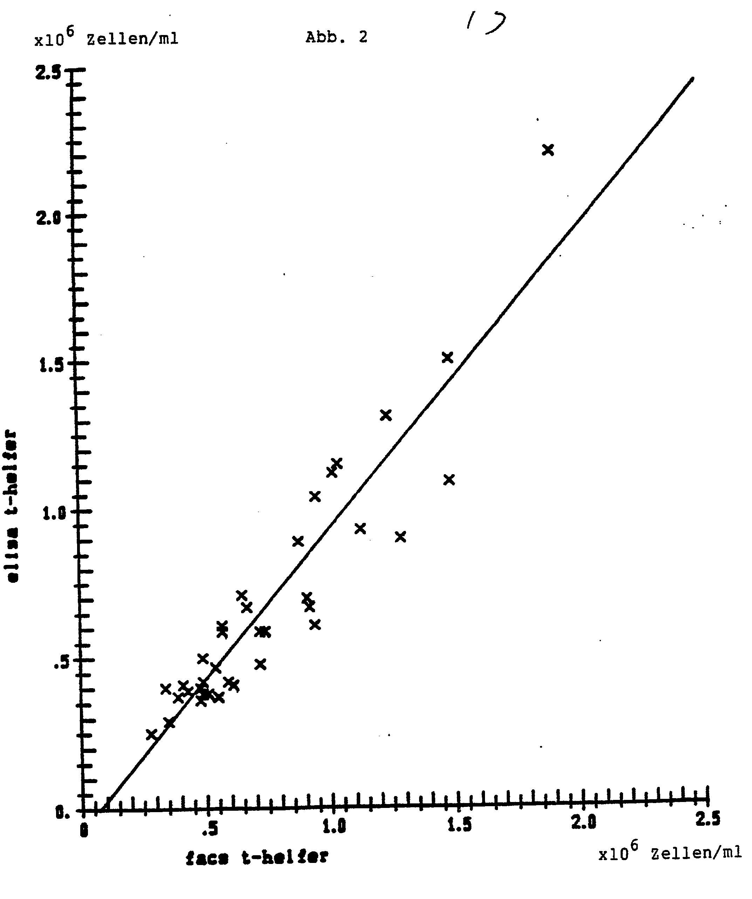

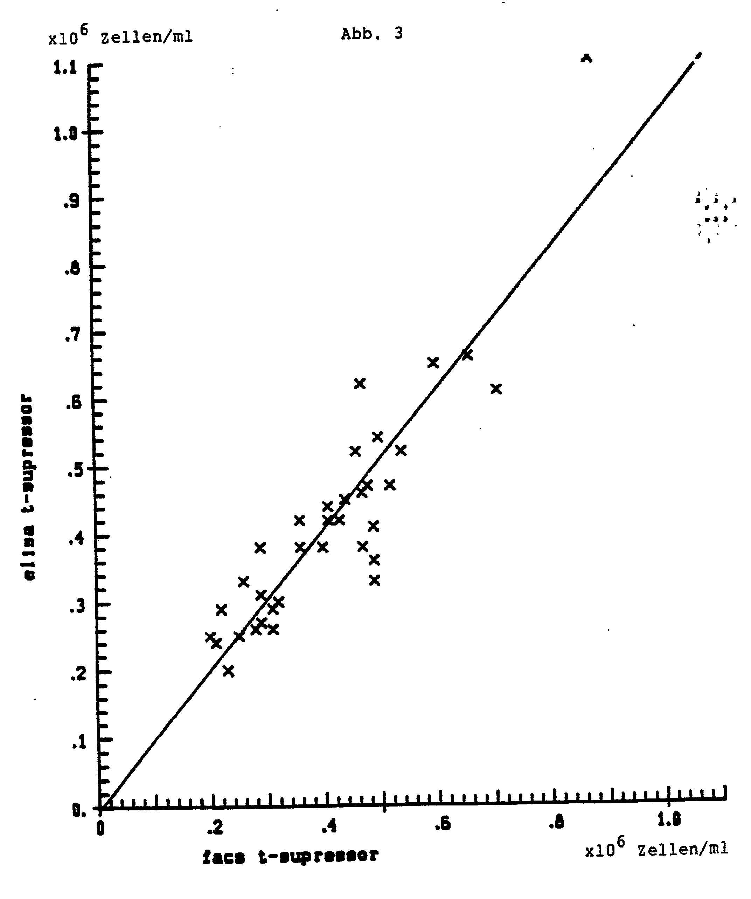

Abb. 1: Methodenvergleich FACS/erfindungsgemäßes Verfahren für Gesamt-T-Lymphozyten (t-pan) Abb. 2: Methodenvergleich FACS/erfindungsgemäßes Verfahren für T-Helfer-Subpopulation (t-helfer) Abb. 3: Methodenvergleich FACS/erfindungsgemäßes Verfahren für T-Suppressor-Subpopulation (t-suppressor)

## Patentansprüche

1. Verfahren zur Quantifizieren einer Zellpopulation bzw. Subpopulation, dadurch gekennzeichnet, daß

die Probe mit der zu bestimmenden Zellpopulation mit markierten Antikörpern inkubiert wird, die spezisch gegen charakteristische Oberflächenantigene der zu quantifizierenden Zellpopulationen gerichtet sind, eine oder mehrere Standards mit bekannten, unterschiedlichen Patikelkonzentrationen mit denselben markierten Antikörpern in gleicher Weise inkubiert werden, wobei die Standardlösung aus Partikeln besteht, die in ihrem Sedimentationsverhalten den zu bestimmenden Zellen gleichen und die mit Molekülen beladen sind, die gegen den markierten Antikörper, bzw. einen Teil hiervon gerichtet sind,

die Zellen der Probelösung sowie die Partikel der Standardlösungen von den überschüssigen markierten Antikörpern abgetrennt werden,

die Menge der Markierung sowohl auf den Zellen als auch auf den Partikeln gemessen wird und

durch Vergleich des Meßwertes aus der Probe mit den Meßwerten aus den Standardlösungen die Anzahl der zu betimmenden Zellen in der Probe ermittelt wird.

2. Verfahren gemäß Anspruch 1, dadurch gekennzeichnet, daß als markierte Antikörper markierte monoklonale Antikörper gegen Oberflächenantigene von T-Lymphozyten bzw. T-Helfer- oder T-Suppressor-Subpopulationen eingesetzt werde.

3. Verfahren gemäß einem der Anspruch 1 oder 2, dadurch gekennzeichnet, daß die Markierung durch Radioisotope, Farbstoffe, Fluoreszenzfarbstoffe oder Enzyme erfolgt.

4. Verfahren gemäß Anspruch 3, dadurch gekennzeichnet, daß als Markierungsenzyme Peroxidase, alkalische Phosphatase, Glukoseoxidase oder β-Galactosidase eingesetzt werden.

5. Standard für die Quantifizierung einer Zellpopulation bzw. Subpopulation, nach einem Verfahren gemäß einem der Ansprüche 1 bis 4, dadurch gekennzeichnet, daß er künstliche Partikel enthält, die in ihrem Sedimentationsverhalten den zu bestimmenden Zellen gleichen und die Moleküle tragen, die gegen den markierten Antikörper bzw. einen Teil hiervon gerichtet sind.

6. Standard gemäß Anspruch 5, dadurch gekennzeichnet, daß die künstlichen Partikel aus Glas oder Kunststoff bestehen.

7. Standard gemäß Anspruch 6, dadurch gekennzeichnet, daß die Kunststoffpartikel aus Latexpartikel auf Methacrylatbasis bestehen.

8. Standard gemäß einem der Ansprüche 5 bis 7, dadurch gekennzeichnet, daß die Partikel Proteine, die dieselbe antigene Wirkung besitzen wie die Oberflächenantigene auf den zu bestimmenden Zellen, Anti-Antikörper, die gegen die markierten Antikörper gerichtet sind, oder Antikörper, welche die Markierung spezifisch erkennen, tragen.

9. Reagenskit zur Durchführung des Verfahrens gemäß einem der Ansprüche 1 bis 4, dadurch gekennzeichnet, daß es den markierten Antikörper, der spezifisch gegen die zu bestimmende Zellpopulation gerichtet ist, einen oder mehrere Standards gemäß einem der Ansprüche 5 bis 8, ein Reagens mit den für die Messung der Markierung erforderlichen Reagenzien sowie gegebenenfalls erforderliche weitere Hilfsmittel enthält.

10. Reagenskit gemäß Anspruch 9, dadurch gekennzeichnet, daß der Antikörper mit β-Galactosidase markiert ist und das Reagens zur Messung der Markierung die für den Nachweis der β-Galactosidase erforderlichen Reagenzien enthält.

# EP 0 224 134 B1

## Claims

1. Process for the quantification of a cell population or sub-population, characterised in that the sample with the cell population to be determined is incubated with labelled antibodies which are specifically directed against characteristic surface antigens of the cell population to be quantified, one or more standards with known, different particle concentrations are incubated in the same way with the same labelled antibodies, whereby the standard solutions consist of particles which, in their sedimentation behaviour, are comparable to the cells to be determined and are loaded with molecules which are directed against the labelled antibodies or a part thereof, the cells of the sample solution, as well as the particles of the standard solutions are separated from the excess labelled antibodies, the amount of the labelling is measured not only on the cells but also on the particles and, by comparison of the measurement value from the sample with the measurement values from the standard solutions, the number of the cells to be determined is ascertained in the sample.

2. Process according to claim 1, characterised in that, as labelled antibodies, there are used labelled monoclonal antibodies against surface antigens of T-lymphocytes or T-helper or T-suppressor sub-populations.

3. Process according to one of claims 1 or 2, characterised in that the labelling takes place by radioisotopes, coloured materials, fluorescence coloured materials or enzymes.

4. Process according to claim 3, characterised in that, as labelling enzyme, there are used alkaline phosphatase, glucose oxidase or β-galactosidase.

5. Standard for the quantification of a cell population or sub-population by a process according to one of claims 1 to 4, characterised in that it contains synthetic particles which are comparable in their sedimentation behaviour to the cells to be determined and which carry molecules which are directed against the labelled antibodies or a part hereof.

6. Standard according to claim 5, characterised in that the synthetic particles consist of glass or synthetic resin.

7. Standard according to claim 6, characterised in that the synthetic resin particles consist of latex particles based on methacrylate.

8. Standard according to one of claims 5 to 7, characterised in that the particles carry proteins which possess the same antigenic action as the surface antigens on the cells to be determined, anti-antibodies which are directed against the labelled antibodies or antibodies which specifically recognise the labelling.

9. Reagent kit for the carrying out of the process according to one of claims 1 to 4, characterised in that it contains the labelled antibody which is specifically directed against the cell population to be determined, one or more standards according to one of claims 5 to 8, a reagent with the reagents necessary for the measurement of the labelling, as well as possibly further necessary auxiliary agents.

10. Reagent kit according to claim 9, characterised in that the antibody is labelled with β-galactosidase and the reagent for the measurement of the labelling contains the reagents necessary for the detection of β-galactosidase.

## Revendications

1. Procédé pour quantifier une population ou sous-population de cellules, caractérisé en ce que:
on fait incuber l'échantillon contenant la population cellulaire à déterminer, avec des anticorps marques, qui sont dirigés spécifiquement contre des antigènes de surfaces caractéristiques de la population cellulaire à quantifier,
on fait incuber de la même façon un ou plusieurs standards, à des concentrations de particules variées, connues, avec le même anticorps marqué, la solution standard étant constituée par des particules dont la propriété de sédimentation est identique à celle des cellules à déterminer, et qui sont chargées de molécules qui sont dirigées contre l'anticorps marqué ou contre une partie de celui-ci,
on sépare les cellules de la solution d'échantillon ainsi que les particules de la solution standard de l'anticorps marqué en excès,
on mesure le degré de marquage aussi bien sur les cellules que sur les particules, et
on détermine le nombre des cellules à déterminer par comparaison entre les résultats des mesures effectuées sur l'échantillon et les résultats des mesures effectuées sur les solutions standards.

2. Procédé selon la revendication 1, caractérisé en ce que comme anticorps marqué, on met en œuvre des anticorps monoclonaux marqués dirigés contre l'antigène de surface de lymphocytes T ou une sous-population de cellules assistantes T ou de suppresseurs T.

3. Procédé selon la revendication 1 ou 2, caractérisé en ce que le marquage s'effectue par radioisotopes, colorants, colorants fluorescents ou enzymes.

4. Procédé selon la revendication 3, caractérisé en ce que comme enzyme de marquage, on utilise les peroxydases, les phosphatases alcalines les glucose-oxydases ou les β-galactosidases.

5. Standard pour la quantification d'une population ou sous-population de cellules, suivant un procédé selon l'une des revendications 1 à 4, caractérisé en ce qu'il contient des particules artificielles, dont la propriété de sédimentation est identique à celle des cellules à déterminer, et qui portent des molécules qui sont dirigées contre l'anticorps marqué ou contre une partie de celui-ci.

EP 0 224 134 B1

6. Standard selon la revendication 5, caractérisé en ce que les particules artificielles sont constituées de verre ou de matière plastique.

7. Standard selon la revendication 6, caractérisé en ce que les particules artificielles sont constituées de particules de latex à base de méthacrylate.

8. Standard selon l'une quelconque des revendications 5 à 7, caractérisé en ce que les particules comportent des protéines présentant la même activité antigénqiue que les antigènes de surface sur les cellules à déterminer, des antianticorps qui sont dirigés contre l'anticorps marqué, ou des anticorps qui reconnaissent spécifiquement le marquage.

9. Kit de réactifs pour la mise en œuvre du procédé selon l'une quelconque des revendications 1 à 4, caractérisé en ce qu'il contient
l'anticorps marqué, qui est dirigé spécifiquement contre la population cellulaire à déterminer,
un ou plusieurs standards selon l'une quelconque des revendications 5 à 8,
un réactif avec les réactifs nécessaires pour la mesure du marquage, ainsi que
d'autres adjuvants éventuellement nécessaires.

10. Kit de réactifs selon la revendication 9, caractérisé en ce que l'anticorps est marqué à la β-galactosidase et que le réactif pour la mesure du marquage contient les réactifs nécessaires pour la détection de la β-galactosidase.

EP 0 224 134 B1

Abb. 1

Abb. 2

Abb. 3